# EUROPÄISCHE PATENTSCHRIFT

(11) **EP 0 535 552 B1**
(45) Veröffentlichungstag und Bekanntmachung des Hinweises auf die Patenterteilung: **02.01.1997**
(21) Anmeldenummer: 92116453.9
(22) Anmeldetag: 25.09.1992
(51) Int. Cl.: A61B 19/00, F21L 15/20

(54) **Stirnreif für eine Mess-, Beleuchtungs- oder Beobachtungseinheit**
Headband for measuring, lighting or viewing device
Bandeau de tête pour dispositif de mesure, d'éclairage ou d'observation

(30) Priorität: 04.10.1991 DE 9112375 U; 04.10.1991 DE 9112376 U; 04.10.1991 DE 9112377 U
(43) Veröffentlichungstag der Anmeldung: 07.04.1993
(73) Patentinhaber: Carl Zeiss, D-89520 Heidenheim (Brenz) (DE); CARL-ZEISS-STIFTUNG, HANDELND ALS CARL ZEISS, D-89518 Heidenheim (Brenz) (DE)
(72) Erfinder: Hildenbrand, Peter, W-7901 Beimerstetten (DE); Gottlob, Heinz, W-7923 Königsbronn (DE); Matuschek, Walter, W-7080 Aalen (DE)

(56) Entgegenhaltungen:
- WO-A-89/02202
- US-A- 3 830 230
- US-A- 4 681 413

## Beschreibung

Die Erfindung betrifft einen Stirnreif mit einer Halterungseinrichtung für eine Meß-, Beleuchtungs- oder Beobachtungseinheit nach dem Oberbegriff des Anspruchs 1. Ein derartiger Stirnreif geht beispielsweise aus der US-A-3 830 230 hervor.

Ein derartiger Stirnreif dient dazu Geräte am Kopf eines Benutzers anzubringen, damit dieser seine beiden Hände für seine Arbeit frei hat und sich das Gerät bei seiner Arbeit immer vor seinen Augen befindet. Ein verbreitetes Anwendungsgebiet des Stirnreifs ist deshalb die Mikrochirurgie, Zahnmedizin und auch die Ophthalmologie.

Die am Markt angebotenen Stirnreife sind mit Innenpolstern aus Kunststoff, Schaumstoff oder Leder ausgestattet. Problematisch bei allen diesen Stoffen ist die Schweißbildung des Stirnreifträgers und der Feuchtigkeitsstau unter diesen Abpolsterungen.

Aus dem DE-GM 83 34 797 ist ein Stirnreif mit einer Beobachtungs- und einer Beleuchtungseinheit bekannt, bei welchem der Stirnreif an der Hinterkopfseite offen ist, wobei die offenen Enden zur Einstellung des Reifdurchmessers übereinanderschiebbar und mit einer Feststellschraube zusammengehalten werden und bei welchem die Einstellschraube als Gegengewicht für die Beobachtungs- und Beleuchtungseinheit gestaltet ist.

Aus der DE-PS 35 16 581 ist ein als Kopfband bezeichneter Stirnreif mit wenigstens einem abnehmbaren Polster an wenigstens einem Abschnitt des Stirnreifs bekannt, bei welchem das Polster aus einem elastischformstabilen Material ausgebildet ist und ein Laschenprofil aufweist, das hakenartig über die Ränder des Stirnreifs greift und daran eingerastet ist.

Aus der DE-PS 33 22 183 und der DE-PS 25 34 784 ist ein als Kopfgestell bezeichneter Stirnreif mit Stellvorrichtung für das Stirnband zur Einstellung auf den individuellen Kopfumfang bekannt, welcher eine Kopfband besitzt.

Sowohl das DE-GM 83 34 797 als auch die DE-PS 25 34 784 und die DE-PS 33 22 183 besitzen am stirnseitigen Ende des Stirnreifs eine Haltevorrichtung für eine Beobachtungseinheit.

Es ist die Aufgabe der Erfindung einen Stirnreif zu schaffen, welcher eine leichte individuelle Nutzung einer Meß-, Beleuchtungs- oder Beobachtungseinheit am Stirnreif bei angenehmen Tragekomfort gestattet.

Diese Aufgabe wird durch den kennzeichnenden Teil des ersten Patentanspruches gelöst. Weitere vorteilhafte Ausgestaltungen der Erfindung sind in den Unteransprüchen beschrieben.

Durch die erfindungsgemäße Anbringung des Bügels am Stirnreif kann das angebrachte Gerät leicht individuell gemäß den Bedürfnissen des Trägers eingestellt werden, ohne daß Abstriche am Tragekomfort hingenommen werden müssen.

Vorteilhafter Weise ist das Stirnband ein seiner Länge veränderbar, was positiv zum Tragekomfort beiträgt.

Vorteilhafter Weise könnte die Beweglichtkeit des Kopfbandes es dem Träger erlauben, das Kopfband dort auf dem Kopf aufliegen zu lassen, wo es für ihn am angenehmsten ist und das Meß-, Beleuchtungs- oder Beobachtungsgerät am Stirnreif durch das Kopfband den größtmöglichen Halt erfährt.

Dieser angenehme Tragekomfort und die sichere Kopfanlage des Stirnreifs wird vorteilhafter Weise durch eine Zweiteilung des Stirnbandes unterstützt, wodurch das hintere Stirnband im Nackenbereich des Trägers zur Anlage kommen kann. Vorteilhafter Weise wird das Stirn- und/oder das Kopfband längenveränderlich gestaltet.

Vorteilhafter Weise könnte eine leichte individuelle Anpassung des Stirnreifs an den Kopf des Trägers durch das Kopfband erzeilt werden, wenn es an seinen beiden Enden beweglich über Verbindungselemente an dem Stirnband befestigt ist.

Vorteilhafter Weise verhindert eine Schutzkappe an der Meß-, Beleuchtungs- oder Beobachtungseinheit deren Verschmutzung bei der Einrichtung des Stirnreifs auf den Kopf des Trägers.

Vorteilhafter Weise ist durch eine klemmende Befestigung der Gerätes deren schneller Austausch sichergestellt (sowie deren leichte Anpassung an unterschiedliche Träger des Kopfbandes).

Die Anbringung der Gerätes an eine spezielle Halterungseinrichtung sichert eine optimale Lageveränderung des Gerätes relativ zu den Blickachsen der Augen des Trägers.

Um eine kurzzeitige, sichere Entfernung der Meß-, Beleuchtungs- oder Beobachtungseinheit vor den Augen des Stirnreif-Trägers ohne Abnahme der Meß-, Beleuchtungs- oder Beobachtungseinheit zu ermöglichen, ist der Bügel, an welchem die Meß-, Beleuchtungs- oder Beobachtungseinheit befestigt ist, an dem Grundkörper mittels einer Rasterhalterung nach oben drehbar gelagert.

Um einen angenehmen Tragekomfort sicherzustellen, könnte im Innenbereich des Stirnreifs im Stirn-, Hinterkopf- und/oder im Schädelbereich des Trägers zumindest eine Polsterung angebracht werden.

Die an dem erfindungsgemäßen Stirnreif anbringbare Polsterung (bzw. Polsterungen) saugen den Schweiß des Stirnreifträgers auf, verhindern somit einen Feuchtigkeitsstau und sorgen so auch bei einer längeren Tragedauer dafür, daß an den Anlegestellen des Stirnreifs am Kopf des Stirnreifträgers der austretende Schweiß nicht zu einer Beeinträchtigung des Tragekomforts führt. Die leichte Abnehmbarkeit stellt dabei zusätzlich sicher, daß bei mit Schweiß vollgesogenen Polsterungen oder aber bei einem Wechsel des Stirnreifträgers schnell wieder hygienische Verhältnisse erzielt werden können. Außerdem erlaubt die leichte Abnehmbarkeit der Polsterungen, daß der Stirnreif bei der Verwendung im medizinischen Bereich vollständig desinfiziert werden kann. Vorteilhafter Weise erreicht man die leichte Abnehmbarkeit der Polsterung dadurch, daß die Polsterung mittels eines Klettbandes am Stirnreif befestigt wird.

Eine Oberflächenhülle sollte zur leichten Schweißaufnahme aus einem wasserdurchlässigen Material bestehen, z.B. Polypropylen oder ein Mischgewebe mit ähnlichen Eigenschaften. Das Material selber sollte dabei hautfreundlich aber wasserabweisend sein. Selbstverständlich sind auch alle anderen wasserdurchlässigen Materialien geeignet, welche eine den oben genannten Stoffen entsprechende Reißfestigkeit und Hautverträglichkeit aufweisen.

In der Polsterung wird vorteilhafter Weise folgend auf die Oberflächenhülle mindestens ein weiterer Stoff angebracht, welcher stark feuchtigkeitsabsorbierend ist (z.B. Viskosenadelfilz, Baumwolle oder ein Material mit entsprechend guten feuchtigkeitsabsorbierenden Eigenschaften).

Indem man die Polsterung auf einem biegeelastischen Träger, welcher sich im Stirn-, Nacken- und/oder Schädeldeckenbereich befinden kann, erreicht man, daß die Polsterung möglichst großflächig auf dem Kopf des Stirnbandträgers aufliegt.

Polsterungen am hinteren, und/oder vorderen Bereich des Stirnbandes und/oder am Kopfband erhöhen den Tragekomfort des Stirnreifs wesentlich.

Eine Befestigung der hinteren Polsterung auf einem biegeelastischen Träger erbringt eine optimale Anlage der Polsterung. Dieser Träger ist vorteilhafter Weise an der Verstellaufnahme befestigt, um einen optimalen Sitz der Polsterung bei jedem Kopfumfang sicherzustellen.

In den nun folgenden Figuren wird die Erfindung anhand von einem Ausführungsbeispiel näher erläutert, wobei dem besseren Verständnis dienende Erläuterungen und Ausgestaltungsmöglichkeiten der Erfindung beschrieben sind.

Es zeigen
- Fig. 1: der Stirnreif in Frontalansicht;
- Fig. 1a: eine Detailansicht der Schwenkmechanik der Befestigungsvorrichtung des Stirnreifs aus Fig. 1;
- Fig. 2: eine Aufsicht auf den Stirnreif aus Fig. 1;
- Fig. 3: eine Seitenansicht des Stirnreifs aus Fig. 1;
- Fig. 3a: eine Detailansicht eines Verbindungselementes zwischen den vorderen und hinteren Teil des Stirnreifs aus Fig. 4;
- Fig. 4: eine seitliche Schnittzeichnung durch den Stirnreif gemäß der Schnittlinie in Fig. 2;
- Fig. 5: einen Griffschutz für eine Prismenlupe und
- Fig. 6: eine Prismenlupe mit aufgesetztem Griffschutz.

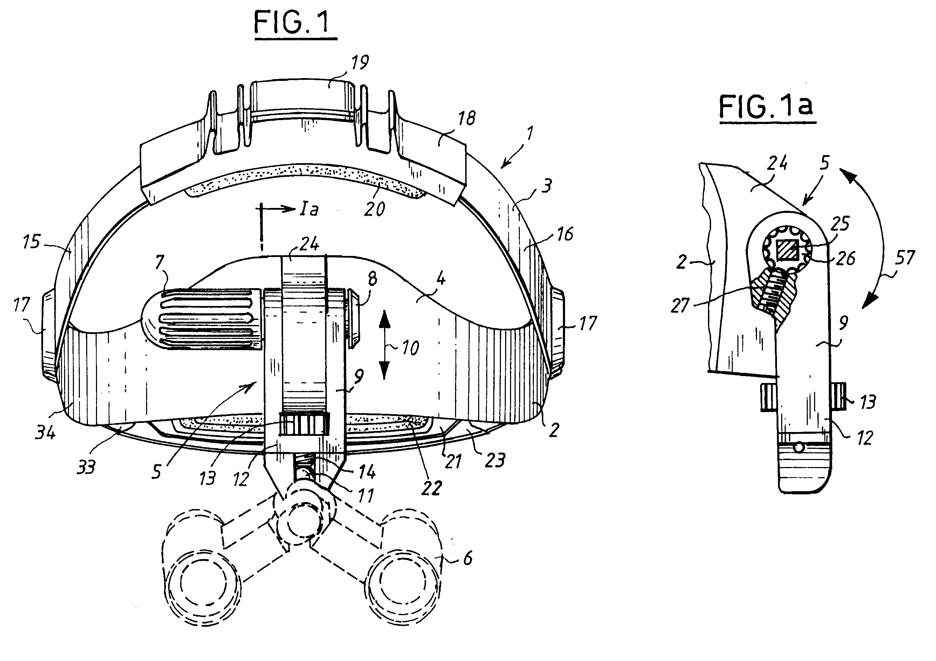

Der in der Figur 1 dargestellte Stirnreif (1) besitzt ein Stirnband (2) und ein Kopfband (3), welches auf dem Kopf eines Benutzers geht. Beide Bänder (2, 3) bestehen aus einem flexiblen Hartplastikmaterial, welches leicht zu reinigen und zu desinfizieren ist. Das Stirnband (2) besitzt an seiner Stirnseite eine Bandverbreiterung (4), an welcher sich eine Halterungseinrichtung (5) für eine Beobachtungseinheit (6) (z.B. eine Prismenlupe wie in der Zeichnung dargestellt) angebracht ist.

Diese Halterungseinrichtung (5) besteht aus einem stabilen Grundkörper (24), an welchem mit einem Stellknopf (7) und einer Spannschraube (8) ein drehbeweglicher Bügel (9) höhenverstellbar entlang der Bewegungslinie (10) angebracht ist. An diesem Bügel (9) befindet sich eine Halterungseinrichtung für die optische Beobachtungseinheit (6).

Sowohl der Grundkörper (24) als auch der Bügel (9), der Stellknopf (7) und die Spannschraube (8) besitzen eine leicht zu desinfizierende Oberfläche (z.B. Kunststoff).

Die Beobachtungseinheit (6) besitzt eine Kugelkopfverlängerung (11) (z.B. aus Stahl), welche bei der Befestigung an den Bügel (9) in eine sich im Endbereich (12) des Bügels (9) befindliche, entsprechend geformte Aushöhlung eingelegt ist. In dieser Aushöhlung wird die Kugelkopfverlängerung (11) durch eine an einem Feststellzahnrad (13) befindliche Gewindeschraube (14) in ihrer Lage fixiert. Die Gewindeschraube (14) geht dabei durch eine Gewindebohrung (in der Zeichnung nicht dargestellt) im Bügelendbereich (12). Löst man mit dem Feststellzahnrad (13) die Gewindeschraube (14), so kann man die Beobachtungseinheit (6) auf einer Kugelfläche mit Mittelpunkt in der Kugel der Kugelkopfverlängerung (11) bewegen und die ausgewählte Stellung der Beobachtungseinheit (6) durch festdrehen der Gewindeschraube (14) mittels des Feststellzahnrades (13) fixieren. Alle Kanten an der Halterungseinrichtung (5) sind abgerundet, um eine Verletzungsgefahr zu minimieren.

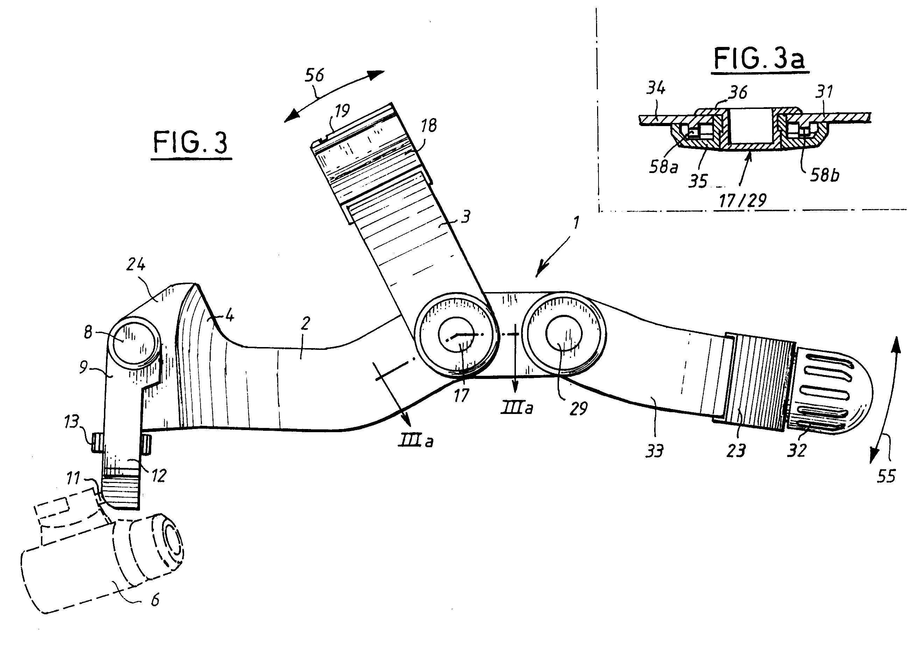

Das Kopfband (3) ist zweigeteilt, wobei der linke und der rechte Kopfbandteil (15, 16) jeweils an einem Ende mittels einem Verbindungselement (17) am vorderen Teil (34) des Stirnbandes (2) so beweglich angeordnet ist, daß eine Bewegung des Kopfbandes (3) entlang der Bewegungslinie (56; siehe Fig. 3) Stirn-Hinterkopf des Stirnreifträgers (hier nicht dargestellt) möglich ist. Die beiden anderen Bandenden der Kopfbandteile (15, 16) ragen in eine Verstellaufnahme (18). Diese Verstellaufnahme (18) ist nur seitlich für das Einschieben der Bandenden der Kopfbandteile (15, 16) offen und umschließt die Kopfbandteile (15, 16) ansonsten vollständig. Auf dem oberen zentralen Teil der Verstellaufnahme (18) befindet sich eine Schiebetaste (19). Durch Verschiebung der Verschiebetaste (19) in Richtung auf die Blickrichtung des Trägers werden die Kopfbandteile (15, 16) in der Verstellaufnahme (18) gelöst und das Kopfband (3) kann geweitet werden. Befindet sich der Stirnreif (1) auf dem Kopf des Trägers, so können die Kopfbandteile (15, 16) rastend nach bekanntem Stand der Technik in die Verstellaufnahme (18) eingeschoben werden, um ein zu großes Kopfband (3) an den Kopf des Trägers anzupassen.

Unterhalb der Verstellaufnahme (18) ist eine Polsterung (20) angebracht, um ein angenehmes Tragen des Stirnbandes (1) sicherzustellen.

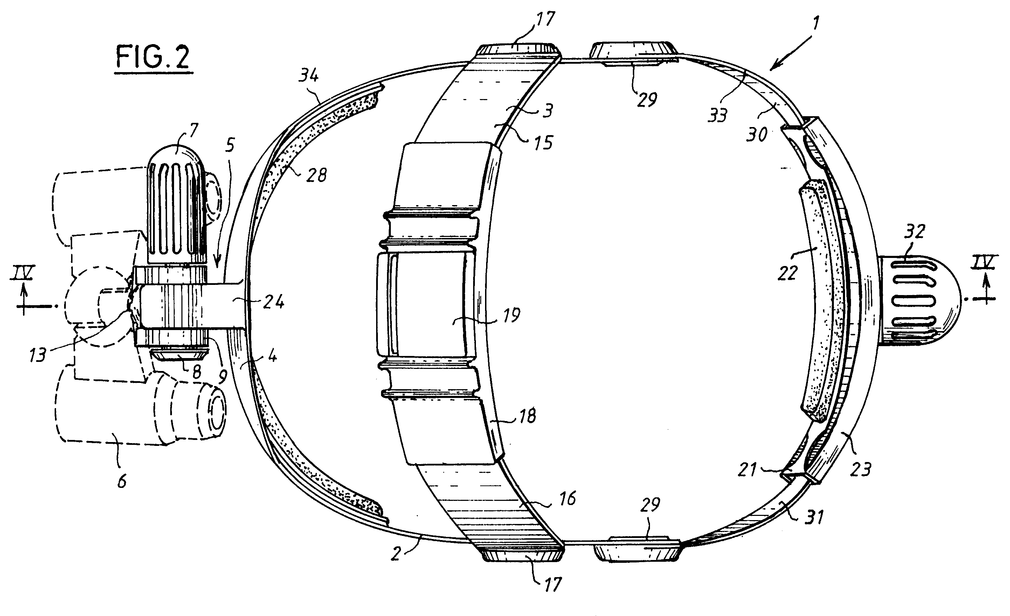

Am hinteren Ende des Stirnbandes (2), im Bereich des Hinterkopfes eines Trägers, besitzt das Stirnband (2) auf der Innenseite einen biegeelastischen Träger (21), auf welchem eine weitere Polsterung (22) angebracht ist. Dieser Träger (21) ist an der Verstellaufnahme (23) des Stirnbandes (2) angebracht. Dies wird in Figur 2 näher erläutert.

In Figur 1a ist die rastende Schwenkmechanik der Halterungseinrichtung (5) dargestellt. Im Bereich des Bügels (9) hat ein Stahlstift (25) an der Spannschraube (8) einen quadratischen Querschnitt. Um den Stahlstift (25) herum ist auf den beiden Seiten des Bügels (9) jeweils eine Rastscheibe (26) angebracht. Mit einem federnden Druckstift (27) werden die Raststellungen fixiert. Durch Ein- oder Herausdrehen dieses Druckstiftes (27) kann man die Kraft zur Veränderung von einer Raststellung zur nächsten einstellen, wobei sich der Bügel (9) entlang der Bewegungslinie (57) bewegen kann.

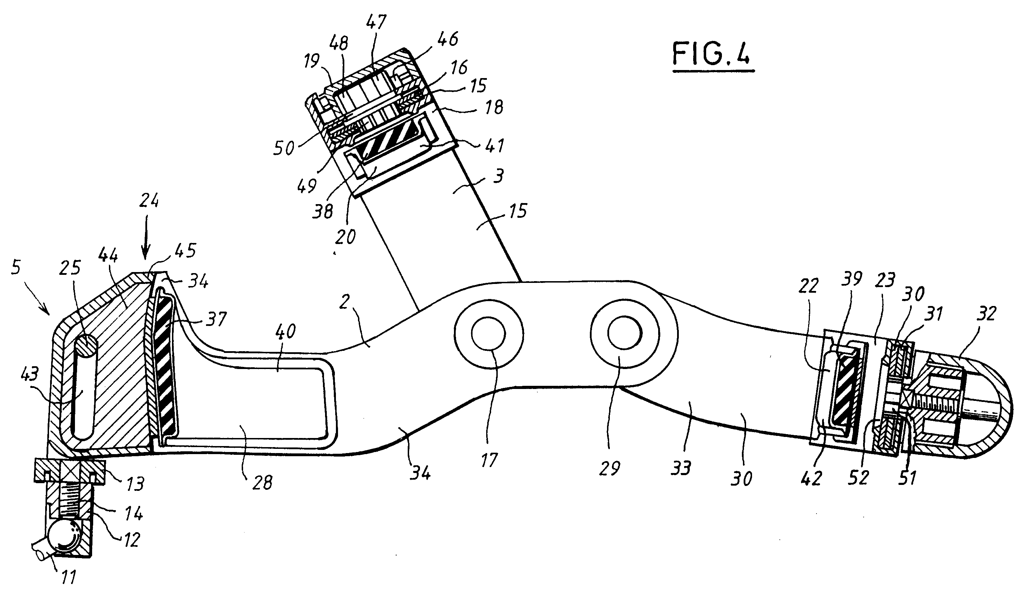

In Figur 2 ist der Stirnreif (1) in Aufsicht dargestellt. Das Stirnband (2) besitzt jeweils eine stirn- und hinterkopfseitige Polsterung (28, 22). Das Kopfband (3) ist mit seinen beiden Teilen (15, 16) endseitig mittels Verbindungselementen (17) am Stirnband (2) befestigt. Die Längenvergrößerung des Kopfbandes (3) erfolgt durch Verschiebung der Verschiebetaste (19) in Richtung auf die Beobachtungseinheit (6). Die Längenverminderung des Kopfbandes (3) erfolgt durch das Hineinschieben der Kopfbandteile (15, 16) in die Verstellaufnahme (18). Die Halterungseinrichtung (5) mit Grundkörper (24), Bügel (9), Stellknopf (7), Spannschraube (8) und Einstellzahnrad (13) an der Bandverbreiterung (4) des vorderen Stirnbandes (34) wird in Figur 4 noch näher erläutert.

Das vordere Stirnband (34) und das hintere Stirnband (33) sind an beiden Enden mit jeweils einem Verbindungselement (29) miteinander verbunden. Dieses Verbindungselement (29) entspricht in seinem Aufbau dem Verbindungselement (17) und ist zu Figur 3a näher erläutert.

Am hinteren Stirnband (33) befindet sich die Verstellaufnahme (23), in welcher durch Drehung der Feststellschraube (32) die Längenanpassung des Stirnbandes (1) an den Kopfumfang des Trägers erfolgt. Um diese Längenanpassung zu ermöglichen, besteht das hintere Stirnband (33) aus zwei Teilen (30, 31). Die hintere Polsterung (22) ist auf einem biegeelastischen Träger (21) angebracht, welcher seinerseits an seinen Enden an der Verstellaufnahme (23) befestigt ist.

Die Seitenansicht des Stirnreifs (1) in Figur 3 zeigt deutlich die ergonomisch günstige Gestaltung des Stirnreifs (1). Der Stirnreif (1) hat im Stirnbereich des Trägers eine Bandverbreiterung (4), um eine sichere Anbringung der Beobachtungseinheit (6) zu gewährleisten. Im Schläfenbereich des Trägers wird das Stirnband (2) leicht nach oben geführt, damit der Stirnreif (1) die Ohren des Trägers nicht stört. Oberhalb der Ohren des Trägers ist der Stirnreif (1) gerade gestaltet. An dem Verbindungselement (29) ist das hintere Stirnband (33) angebracht. Dieses hintere Stirnband (33) kann auf der Bewegungslinie (55) nach oben oder unten bewegt werden und damit der Stirnreif (1) hinsichtlich Tragekomfort und Sicherheit optimal ausgerichtet werden. Die Anpassung der Länge des Kopfbandes (3) und des Stirnbandes (2) erfolgt dann oder gleichzeitig mit der Ausrichtung des Stirnbandes (2) in den Verstellaufnahmen (18, 23).

Figur 3a zeigt die einfache, aber sehr sichere Konstruktion der Verbindungselemente (17, 29) am Beispiel der Stirnband-Befestigung. An den Enden des vorderen und des hinteren Stirnbandes (34, 31) sind Erhebungen (58a, b) ausgebildet. Diese Erhebungen (58) greifen in entsprechende radiale Aussparungen eines äußeren Deckels (35) des Verbindungselementes (29). Zur Fixierung des äußeren Deckels (35) wird in diesen ein innerer Deckel (36) eingeklippt, wobei der innere Deckel (36) an seiner freien Außenseite eine lange radialförmige Verlängerung (36) aufweist. Diese Verlängerung (36) stellt sicher, daß die Stirnbänderteile (34, 31) nicht aus dem Verbindungselement (29) gleiten. Das Verbindungselement (17) für die Befestigung des Kopfbandes (3) am Stirnband (2) ist entsprechend aufgebaut. Dieses Verbindungselement (17, 29) erlaubt eine sichere, dauerhafte und trotzdem preiswerte Verbindung zwischen Plastikteilen begrenzter Elastizität.

Im Querschnitt der Figur 4 zeigt sehr deutlich den Aufbau der Verstellaufnahme (18, 23) und die innere Gestaltung der Halterungseinrichtung (5).

Die am vorderen Stirnbandteil (34) angebrachte Halterungseinrichtung (5) besteht aus dem Grundkörper (24), welcher aus einem Innenkörper (44) (z.B. aus Leichtmetall oder glasfaserverstärktem Kunststoff) mit einer dicken Hartplastikumhüllung (45) besteht. In dem Grundkörper (24) befindet sich ein Langloch (43), durch welches ein Stahlstift (25) als Verlängerung der Spannschraube (8) in seinem Bewegungsspielraum begrenzt wird. An diesem Stahlstift (25) ist der Stellknopf (7) über ein Gewinde (in dieser Figur nicht dargestellt) befestigt. Zwischen Stellknopf (7) und Spannschraube (8) befindet sich seitlich um den Grundkörper (24) ein Bügel (9) (z.B. aus Aluminium) mit einem Loch (in der Zeichnung nicht dargestellt), durch welches der an der Spannschraube (8) befindliche Stahlstift (25) geht. Verringert man die durch den Stellknopf (7) und die Spannschraube (8) bewirkte Klemmspannung, so kann man den Bügel (9) relativ zum Grundkörper (24) in vertikaler Richtung verschieben. Hat die Beobachtungseinheit (6) die richtige Höhenlage relativ zu den Augen des Benutzers (in der Zeichnung nicht dargestellt), so kann man die vertikale Relativlage des Bügels (9) zum Grundkörper (24) durch festdrehen an dem Stellknopf (7) fixieren. Die Vertikalverschiebung ist dabei durch das Längsloch (43) begrenzt. Der Stellknopf (7) ist in bekannter Art und Weise vor Abdrehen von dem Gewinde des Stahlstiftes (25) der Spannschraube (8) lösbar geschützt, so daß bei einer Veränderung der vertikalen Höhenlage der Beobachtungseinheit (6) nie die Gefahr besteht, daß die Beobachtungseinheit (6) sich unkontrolliert von dem Stirnreif (1) löst und damit zerstört wird oder eine Zerstörung anrichtet. Im unteren Bereich des Bügels (9) befindet sich das Feststellzahnrad (13) mit der Gewindeschraube (14), mit welcher die Lage der Kugelkopfverlängerung (11) fixiert wird. Die stirnseitig angeordnete Polsterung (28) (z.B. aus Schaumstoff) besitzt eine rutschfeste Umhüllung (40) (z.B. aus Silikon) um eine Innenpolsterung (37).

Am vorderen Stirnbandteil (34) ist das Kopfband (3) mit einem Verbindungselement (17) drehbar befestigt. Unter der Verstellaufnahme (18) befindet sich eine Polsterung (20), welche sich aus einer Innenpolsterung (38) (z.B. Schaumstoff) und einer rutschfesten Umhüllung (41) (z.B. aus Silikon) aufgebaut ist. Auf der Verstellaufnahme (18) befindet sich eine Schiebetaste (19) zur Lösung einer Rutscharretierung (46) eines Zahnrades (47). Wird die Schiebetaste (19) bewegt, so wird die Rutscharretierung (46) gelöst, die beiden Teile (15, 16) des Kopfbandes (3) können sich aus der Verstellaufnahme (18) hinausbewegen und damit kann das Kopfband (3) bis zu seiner größten Länge vergrößert werden. Ohne Verschiebung der Schiebetaste (19) lassen sich die beiden Kopfbandteile (15, 16) in die Verstellaufnahme (18) hineinschieben. Das Zahnrad (47) besitzt einen oberen und einen unteren Zahnkranz (48, 49), welche durch eine, eine Rotationsbewegung ermöglichende Halterungsscheibe (50) getrennt sind. Die beiden Kopfbandteile (15, 16) sind bezogen auf den unteren Zahnkranz (49) mit entsprechenden, passenden Einkerbungen versehen, welche auf den sich gegenüberliegenden Seiten in den Zahnkranz (49) fassen. Bei einer Verschiebung eines Kopfbandteiles (15, 16) wird gleichzeitig eine entsprechende Verschiebung des anderen Kopfbandteiles (16, 15) bewirkt.

Das hintere Kopfbandteil (33) ist am vorderen Kopfbandteil (34) mit einem Verbindungselement (29) drehbar befestigt. An diesem hinteren Kopfbandteil (33) befindet sich in der Nackenregion des Trägers eine Verstellaufnahme (23), mittels welcher der Durchmesser des Kopfbandes (2) an die Kopfform des Trägers angepaßt werden kann. Um diese Anpassung vornehmen zu können, ist an der Verstellaufnahme (23) eine Feststellschraube (32) drehbar angebracht, welche durch eine drehbar gelagerte Gegenplatte (52) an der Verstellaufnahme (23) gehalten wird. Durch Drehung der Feststellschraube (32) wird ein Zahnkranz (51) mitbewegt. Die beiden hinteren Stirnbandteile (30, 31) sind bezogen auf diesen Zahnkranz (51) mit entsprechenden passenden Einkerbungen versehen, welche auf den sich gegenüberliegenden Seiten in den Zahnkranz (51) fassen. Bei einer Drehung der Feststellschraube (32) kommt es somit zu einer entsprechenden, gemeinsamen Verschiebung beider hinteren Kopfbandteile (30, 31). Unterhalb der Verstellaufnahme (23) ist eine Polsterung (22), welche aus einer Innenpolsterung (39) (z.B. aus Schaumstoff oder Viskosenadelfilz) mit einer rutschfesten Umhüllung (42) (z.B. aus Silikon oder Baumwolle/Polypropylen) besteht, angebracht.

Die in Figur 4 dargestellten Polsterungen (20, 22, 28) haben einen einheitlichen Aufbau. Ihr besonderer Vorteil besteht darin, daß sie sehr gut den Schweiß des Stirnbandträgers aufnehmen, somit einen Feuchtigkeitsstau verhindern und sehr leicht vom Stirnreif abgenommen werden könne.

Auf dem Stirnreif (1) selber befindet sich an den Stellen, an denen sich die Polsterungen (20, 22, 28) befinden, der härtere Teil eines Klettbandes aufgeklebt. (Die Befestigung dieses Klettbandteiles kann aber auch mit allen anderen bekannten Befestigungsmethoden, z.B. festschrauben erfolgen.) Der andere, weichere Teil des Klettbandes, auch Klettflausch genannt (Dicke ungefähr 1,5 mm) ist an einer gut schweißdurchlassenden Oberflächenhülle (40, 41, 42) aus Polypropylen oder ein Stoff mit ähnlichen Materialeigenschaften angenäht (wahlweise mit diesem an den Seiten verklebt oder nach einem anderen bekannten Stand der Technik, z.B. anheften, mit diesem verbunden). Auf der hautabgewandten Seite der Oberflächenhülle (40, 41, 42) befindet sich eine Baumwollschicht. Diese Schicht sollte frottiert sein. Die Dicke der Oberflächenhülle (40, 41, 42) beträgt ungefähr 1,5 mm.

In der Polsterung (20, 22, 28) befindet sich eine Innenpolsterung (37, 38, 39) aus Viskosenadelfilz (Dicke ungefähr 2 mm) (oder einem anderen Material mit entsprechenden Eigenschaften), welches zum Aufsaugen der durch die Oberflächenhülle (40, 41, 42) dringenden Feuchtigkeit dient.

Durch diesen konstruktiven Aufbau der Polsterungen (20, 22, 28) erreicht man, daß diese sogar waschbar sind und damit mehrfach verwendet werden können.

Die angegebenen Dicken der Polsterungsschichten sind beispielhaft und sollen auf das Bewicht des vorne anzubringenden Gerätes (6) abgestimmt sein.

Die Bandenden (in der Figur nicht dargestellt) der Kopfbandteile (15, 16) sind so gestaltet, daß die Kopfbandteile (15, 16) bei verschobener Verschiebetaste (19) sich nicht unkontrolliert (d.h. ohne bewußten Lösungswillen) aus der Verstellaufnahme (18) entfernen können. Bei dem Einschieben der Kopfbandteile (15, 16) in die Verstellaufnahme (18) ist durch die Gestaltung der Kopfbandteile (15, 16) und der Rastvorrichtung (46) unterhalb der Schiebetaste (19) sichergestellt, daß die beiden Kopfbandteile (15, 16) sich immer mit der gleichen Geschwindigkeit in die Verstellaufnahme (18) bewegen, so daß die Verstellaufnahme (18) sich immer in der Mitte des Kopfbandes (3) befindet. Dadurch liegt das sich unter der Verstellaufnahme (18) angebrachte Polsterung (20) immer ideal auf dem Kopf des Trägers auf und kann einen Teil des Gewichtes der Beobachtungseinheit (6) aufnehmen.

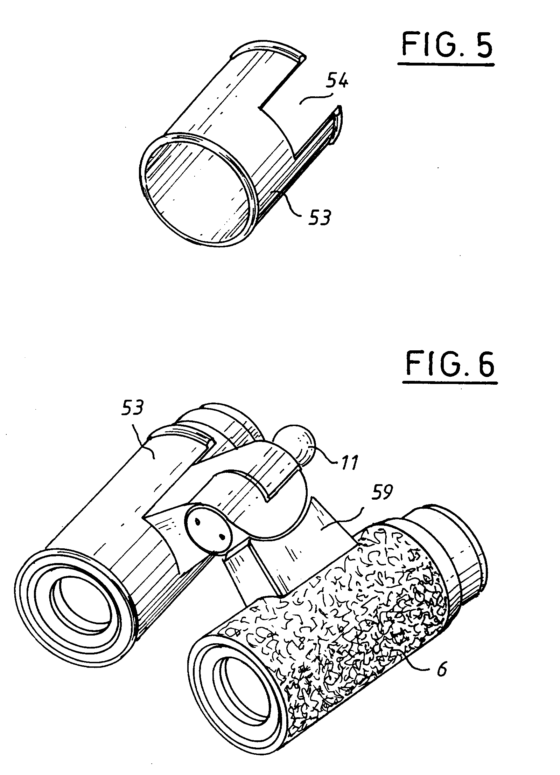

In Figur 5 und 6 ist eine Schutzkappe (53) aus Plastik dargestellt, welche auf eine fernrohrartige Beobachtungseinheit (6) mit zwei getrennten Optiken aufgeschoben werden kann. Diese Schutzkappe (53) ist rohrförmig und besitzt im Bereich der Brücke (59) der Beobachtungseinheit (6) eine Aussparung (54). Die Schutzkappe (53) erlaubt eine Manipulation an der Beobachtungseinheit (6) ohne diese berühren zu müssen. Dies ist insbesondere bei einigen medizinischen Anwendungen, bei welchen es um absolute Sterilität geht, sehr wichtig.

Die Hartplastikumhüllung und das Stirnband können vorteilhafter Weise aus einem Spritzgußteil ausgeführt sein.

## Patentansprüche

1. Stirnreif (1) mit einer Halterungseinrichtung (5) für eine Meß-, Beleuchtungs- oder Beobachtungseinheit (6), mit einem Kopfband (3), welches auf dem Kopf eines Benutzers aufliegend ist, und einem Stirnband (2), bei welchem am Stirnband (2) ein Grundkörper (24) als Teil der Halterungseinrichtung (5) befestigt ist, bei welchem zwischen dem Grundkörper (24) und einem Bügel (9) eine Rasterhalterung angebracht ist und bei welchem an der Rasterhalterung ein Stellknopf (7) angebracht ist, dadurch gekennzeichnet, daß der Bügel (9) relativ zum Grundkörper (24) nach oben durch die Rasterhalterung rastend drehbar gelagert ist, wenn der Stellknopf (7) festgezogen ist, und daß der Bügel (9) zusammen mit der Rasterhalterung an dem Grundkörper (24) in vertikaler Richtung (10) stufenlos beweglich gelagert ist, wenn der Stellknopf (7) gelöst ist.

2. Stirnreif nach Anspruch 1, dadurch gekennzeichnet, daß das Stirnband (2) im Hinterkopfbereich seines Trägers eine Verstellaufnahme (23) angebracht ist, in welche zwei Enden (30, 31) des Stirnbandes (2) hineinragen und daß über eine Feststellschraube (32) das Stirnband (2) längenverstellbar ist.

3. Stirnreif nach Anspruch 2, dadurch gekennzeichnet, daß das Kopfband (3) an seinen beiden Enden über Verbindungselemente (17) an dem Stirnband (2) befestigt ist und gegenüber diesem radial beweglich ist.

4. Stirnreif nach einem der Ansprüche 1 - 3, dadurch gekennzeichnet, daß das Stirnband (2) in ein vorderes Stirnbandteil (34) und mindestens ein hinteres Stirnbandteil (33) unterteilt ist, welche an den jeweiligen Enden der Stirnbandteile (33, 34) über Verbindungselemente (29) radial gegeneinander bewegt werden können.

5. Stirnreif nach Anspruch 4, dadurch gekennzeichnet, daß das hintere Stirnbandteil (33) aus zwei Teilen (30,31) aufgebaut ist, daß die freien Enden der Stirnbandteile (30, 31) im Bereich des Hinterkopfes eines Trägers in einer Verstellaufnahme (23) eingeführt sind und daß die Länge des Stirnbandes (2) mittels einer Feststellschraube (32) an der Verstellaufnahme (23) einstellbar ist.

6. Stirnreif nach einem der Ansprüche 1 - 5, dadurch gekennzeichnet, daß das Kopfband (3) aus zwei Teilen (15, 16) aufgebaut ist, daß die freien Enden der Kopfbandteile (15, 16) im Bereich des Hinterkopfes eines Trägers in einer Verstellaufnahme (16) geführt sind und daß die Länge des Stirnbandes (2) mittels einer Verschiebetaste (18) und bzw. oder einer Rutscharretierung (46) an bzw. in der Verstellaufnahme (16) einstellbar ist.

7. Stirnreif nach einem der Ansprüche 1 - 6 , dadurch gekennzeichnet, daß an einer Meß-, Beleuchtungs- oder Beobachtungseinheit (6) mindestens eine leicht zu desinfizierende Schutzkappe (53) dort angebracht ist, an welcher die Meß-, Beleuchtungs- oder Beobachtungseinheit (6) bei einer Justierung vor dem oder den Augen eines Stirnreifträgers angefaßt wird.

8. Stirnreif nach einem der Ansprüche 1 - 7, dadurch gekennzeichnet, daß an dem unteren Ende der Halterungseinrichtung (5) eine lösbare Klemmeinrichtung für eine Kugelkopfverlängerung (11) einer Meß-, Beleuchtungs- oder Beobachtungseinheit (6) angebracht ist.

9. Stirnreif nach Anspruch 5, dadurch gekennzeichnet, daß ein biegeelastische Träger (21) an der Verstellaufnahme (23) des hinteren Stirnbandes (33) befestigt ist.

10. Stirnreif nach einem der Ansprüche 1 - 9, dadurch gekennzeichnet, daß der Grundkörper (24) im Stirnbereich des Stirnreif-Trägers angebracht ist.

11. Stirnreif nach einem der Ansprüche 1 - 10, dadurch gekennzeichnet, daß im Innenbereich des Stirnreifs (1) im Stirn-, Hinterkopf- und/oder im Schädelbereich des Stirnreifträgers eine Polsterung (20, 22, 28) angebracht ist, welche eine leichte Feuchtigkeitsaufnahme gewährleistet, somit einen Feuchtigkeitsstau verhindert und welche leicht vom Stirnreif (1) abnehmbar ist.

12. Stirnreif nach Anspruch 11, dadurch gekennzeichnet, daß die Rückseite der Polsterung (20, 22, 28) aus dem weichen Teil eines Klettbandes besteht und daß der härtere Teil des Klettbandes am Stirnreif (1) befestigt ist.

13. Stirnreif nach Anspruch 11 oder 12, dadurch gekennzeichnet, daß die Oberflächenhülle (40, 41, 42) der Polsterung (20, 22, 28) aus einem feuchtigkeitsdurchlässigen Material besteht.

14. Stirnreif nach Anspruch 13, dadurch gekennzeichnet, daß die Oberflächenhülle (40, 41, 42) aus Polypropylen besteht.

15. Stirnreif nach einem der Ansprüche 11 - 14, dadurch gekennzeichnet, daß in der Polsterung (20, 22, 28) eine Innenpolsterung (37, 38, 39) eingebracht ist, welche feuchtigkeitsabsorbierend ist.

16. Stirnreif nach Anspruch 15, dadurch gekennzeichnet, daß die Innenpolsterung (37, 38, 39) aus Viskosenadelfilz besteht.

17. Stirnreif nach einem der Ansprüche 11 - 16, dadurch gekennzeichnet, daß die Polsterung (20, 22, 28) auf einem biegeelastischen Träger (21) nach Anspruch 9 angebracht ist.

18. Stirnreif nach Anspruch 17, dadurch gekennzeichnet, daß an dem Stirnreif (1) mindestens eine Vorrichtung zur Veränderung der Länge des Stirnbandes (2) und/oder des Kopfbandes (3) und an dieser Vorrichtung der biegeelastische Träger (21) angebracht ist.

19. Stirnreif nach einem der Ansprüche 17 oder 18, dadurch gekennzeichnet, daß der biegeelastische Träger (21) für eine Polsterung (22) am hinteren Stirnbandbereich (33) im Bereich des Nackens eines Stirnreif-Trägers angebracht ist.

20. Stirnreif nach einem der Ansprüche 1 bis 19, dadurch gekennzeichnet, daß der Bügel (9) an dem Grundkörper (24) stufenlos drehbar beweglich gelagert ist, wenn der Stellknopf (7) gelöst ist.

## Claims

1. A headgear (1) comprising a holding device (5) for a measuring, illuminating or viewing unit (6), a headband (3) to be worn on the head of a wearer and a frontal band (2) at which a base body (24) as a part of a holding device (5) is connected, said headgear (1) having between the base body (24) and a bracket (9) a latch mounting with adjusting knob (7), characterized in that the bracket (9) is rotatably upwardly latchable held relative to the base body (24) by the latch mounting if the adjusting knob (7) is fixed and that the bracket (9) together with the latch mounting is continuously movable at the base body (24) in vertical direction (10) if the adjusting knob (24) is slacked.

2. The headgear of claim 1, characterized in that the frontal band (2) having an adjusting receptacle (23) in the region of the back of the head of the wearer for the two ends (30, 31) of the frontal band (2) and that by using a fixing screw (32) with this adjusting receptacle (23) it can be adjusted the overall length of the head band (2).

3. The headgear of claim 2, characterized in that at the both ends of the head band (3) are attached to the frontal band (2) radial movable by connecting elements (17).

4. The headgear as claimed in any one of the claims 1 to 3, characterized in that the frontal band (2) being subdivided into a forward part (34) and at least a rearward part (33) which are connected at there respective ends radial movable to each other with connecting means (29).

5. The headgear of claim 4, characterized in that the rearward frontal band part (33) having two segments (30, 31), that the free end portions of the frontal band parts (33) in the region of the back of the head of the wearer are inserted in an adjusting receptacle device (23) and that the length of the frontal band (2) is adjustable using a fixing screw (32) at the adjusting receptacle device (23).

6. The headgear as claimed in any one of the claims 1 to 5, characterized in that the head band (3) having two parts (15, 16), that the free end portions of the head band parts (15, 16) in the region of the back of the head of the wearer are inserted in an adjusting receptacle (16) and that the length of the frontal band (2) is adjustable using a slider (18) and respectively or a latching device (46) at respectively inside the adjusting receptacle (16).

7. The headgear as claimed in any one of the claims 1 to 6, characterized in that at a measuring, illuminating or viewing unit (6) at least an easily disinfectable protective cap (53) is placed where the measuring, illuminating or viewing unit (6) is manually grasped for adjusting in front of the eyes of a wearer of the headgear.

8. The headgear as claimed in any one of the claims 1 to 7, characterized in that at the lower end of the holding device (5) there is a resolvable clamping means for a spherical-head extension (11) of the measuring, illuminating or viewing unit (6).

9. The headgear of claim 5, characterized in that an elastically bendable carrier (21) is mounted at the adjusting receptacle (23) of the rear frontal band (33).

10. The headgear as claimed in any one of the claims 1 to 9, characterized in that the base body (24) is located in the forehead region of the headgear wearer.

11. The headgear as claimed in any one of the claims 1 to 10, characterized in that in the inner part of the headgear (1) there is a padding (20, 22, 28) in the forehead, back and/or skull of the headgear wearer which allow an easy absorption of humidity to prevent a banked-up humidity and which is easily removable from the headgear (1).

12. The headgear of claim 11, characterized in that the rear of the padding (20, 22, 28) is the soft part of a VELCRO band and the hard part of the VELCRO band is attached to the headgear (1).

13. The headgear as claimed in any one of the claims 11 or 12, characterized in that the surface casing (40, 41, 42) of the padding (20, 22, 28) being made of a humidity permeable material.

14. The headgear of claim 13, characterized in that the surface casing (40, 41, 42) being made of polypropylene.

15. The headgear as claimed in any one of the claims 11 to 14, characterized in that inside the padding (20, 22, 28) there is a inner padding (37, 38, 39) which absorb humidity.

16. The headgear of claim 15, characterized in that the material of the inner padding (37, 38, 39) being made of viscose needlefelt.

17. The headgear as claimed in any one of the claims 11 to 16, characterized in that the padding (20, 22, 28) is interposed on an elastically bendable carrier (21) according claim 9.

18. The headgear of claim 17, characterized in that there is at least one device at the headgear (1) to change the length of the frontal band (2) and/or the head band (3) and the elastically bendable carrier (21) is connected with this device.

19. The headgear of claim 17 or 18, characterized in that the elastically bendable carrier (21) for a padding (22) is attached in the rear part of the frontal band (33) at the neck region of a wearer.

20. The headgear as claimed in any one of the claims 1 to 19, characterized in that the bracket (9) is continuously rotatable connected with the base body (24) if the adjusting knob (7) is slacked.

## Revendications

1. Support frontal (1) composé d'un dispositif de fixation (5) recevant une unité de mesure, d'éclairage ou d'observation (6), d'un ruban pariétal (3) reposant sur la tête de l'utilisateur et d'un serre-tête (2), sur lequel est monté un corps de base faisant partie dudit dispositif de fixation (5), un support à crans d'arrêt étant aménagé entre ledit corps de base (24) et un bras (9) et un bouton de réglage étant disposé sur ledit support à crans d'arrêt, caractérisé en ce que le bras (9) est monté de manière à pouvoir être tourné vers le haut relativement au corps de base (24) par incréments dû au support à crans d'arrêt lorsque le bouton de réglage (7) est serré et en ce que le bras (9) peut être déplacé, conjointement avec le support à crans d'arrêt, sur le corps de base (24) en direction verticale (10) de manière continue lorsque le bouton de réglage (7) est desserré.

2. Support frontal selon la revendication 1, caractérisé en ce que le serre-tête (2) porte dans la région occipitale du porteur une monture de réglage (23), dans laquelle s'engagent deux segments (30, 31) du serre-tête (2) et que ledit serre-tête (2) est réglable en longueur par l'intermédiaire d'une vis de fixation (32).

3. Support frontal selon la revendication 2, caractérisé en ce que le ruban pariétal (3) est rélié à ses deux extrémités au serre-tête (2) par l'intermédiaire d'éléments de fixation (17) et en ce qu'il est mobile radialement par rapport à celui-ci.

4. Support frontal selon l'une des revendications 1 - 3, caractérisé en ce que le serre-tête (2) est subdivisé en un ruban frontal (34) et au moins un ruban occipital (33) qui sont rendus mobiles radialement l'un par rapport à l'autre par des éléments de fixation (29) au niveau de leurs extrémités respectives.

5. Support frontal selon la revendication 4, caractérisé en ce que le ruban occipital (33) se compose de deux segments (30, 31), que les extrémités dégagées desdits segments (30, 31) sont insérées dans une monture de réglage (23) se trouvant à l'arrière de la tête du porteur et que la longueur du serre-tête (2) est réglable au moyen d'une vis de réglage (32) disposée sur la monture de réglage (23).

6. Support frontal selon l'une des revendications 1 - 5, caractérisé en ce que le ruban pariétal (3) se compose de deux segments (15, 16), que les extrémités dégagées desdits segments (15, 16) sont insérées dans une monture de réglage (16) se trouvant à l'arrière de la tête du porteur et que la longueur du serre-tête (2) est réglable au moyen d'une touche coulissante (18) et/ou un frein à frottement (46) aménagés sur/dans la monture de réglage (16).

7. Support frontal selon l'une des revendications 1 - 6, caractérisé en ce qu'au moins une pièce de protection (53) facile à désinfecter est adaptée à l'unité de mesure, d'éclairage ou d'observation (6), à l'endroit où on touche cette unité de mesure, d'éclairage ou d'observation (6) au moment de son ajustement devant le ou les yeux d'un porteur du support frontal.

8. Support frontal selon l'une des revendications 1 - 7, caractérisé en ce qu'un dispositif de blocage desserrable destiné à la rallonge à tête sphérique (11) d'une unité de mesure, d'éclairage ou d'observation (6) est adapté à l'extrémité inférieure du dispositif de fixation (5).

9. Support frontal selon la revendication 5, caractérisé en ce qu'un support présentant une bonne élasticité de flexion (21) est monté sur la monture de réglage (23) du ruban occipital (33).

10. Support frontal selon l'une des revendications 1 - 9, caractérisé en ce que le corps de base (24) est disposé dans la région frontale du porteur du support frontal.

11. Support frontal selon l'une des revendications 1 - 10, caractérisé en ce que des coussins (20, 22, 28) sont amenagés à l'intérieur du support frontal (1), dans les régions frontale, occipitale et/ou pariétale, coussins qui garantissent une bonne absorption de l'humidité, empêchant ainsi toute accumulation d'humidité, et qui peuvent être détaches facilement dudit support frontal.

12. Support frontal selon la revendication 11, caractérisé en ce que la face arrière des coussins (20, 22, 28) est constituée par la partie molle d'une fermeture Velcro et que la partie dure de cette fermeture Velcro est montée sur le support frontal (1).

13. Support frontal selon la revendication 11 ou 12, caractérisé en ce que l'enveloppe (40, 41, 42) des coussins (20, 22, 28) est constituée par un matériau perméable à l'humidité.

14. Support frontal selon la revendication 13, caractérisé en ce que l'enveloppe (40, 41, 42) est faite de polypropylène.

15. Support frontal selon l'une des revendications 11 - 14, caractérisé en ce les coussins (20, 22, 28) comprennent un rembourrage intérieur (37, 38, 39) qui absorbe l'humidité.

16. Support frontal selon la revendication 15, caractérisé en ce que le rembourrage intérieur (37, 38, 39) est constitué par du feutre tissé de viscose.

17. Support frontal selon l'une des revendications 11 - 16, caractérisé en ce les coussins (20, 22, 28) sont adaptés à un support présentant une bonne élasticité de flexion (21) selon la revendication 9.

18. Support frontal selon la revendication 17, caractérisé en ce que le support frontal (1) porte au moins un dispositif servant à régler la longueur du serre-tête (2) et/ou du ruban pariétal (3) et que le support présentant une bonne élasticité de flexion (21) est monté sur ce dispositif.

19. Support frontal selon l'une des revendications 17 et 18, caractérisé en ce que le support présentant une bonne élasticité de flexion (21) est destiné à porter un coussin (22) dans la région postérieure du ruban occipital située en face de la nuque du porteur du support frontal.

20. Support frontal selon l'une des revendications 1 - 19, caractérisé en ce le bras (9) peut être déplacé en continu par une rotation par rapport au corps de base (24) lorsque le bouton de réglage (7) est desserré.
